# EUROPEAN PATENT SPECIFICATION

(11) **EP 1 143 985 B1**
(45) Date of publication and mention of the grant of the patent: **25.07.2007**
(21) Application number: 00900788.1
(22) Date of filing: 17.01.2000
(51) Int. Cl.: A61K 35/74, A61P 29/00

(54) **USE OF LACTOBACILLUS SALIVARIUS**
VERWENDUNG VON LACTOBACILLUS SALIVARIUS
UTILISATION DE (LACTOBACILLUS SALIVARIUS)

(30) Priority: 15.01.1999 IE 990033; 20.09.1999 IE 990782
(43) Date of publication of application: 17.10.2001
(73) Proprietor: Enterprise Ireland (trading as BioResearch Ireland), Dublin 9 (IE); University College Cork-National University of Ireland, Cork, Cork (IE)
(72) Inventor: COLLINS, John Kevin, Doughcloyne, County Cork (IE); O'SULLIVAN, Gerald Christopher, Bishopstown, Cork (IE); O'MAHONY, Liam, Ballintemple, Cork (IE); SHANAHAN, Fergus, Kinsale, County Cork (IE)
(74) Representative: O'Brien, John Augustine
(86) International application number: PCT/IE2000/000007
(87) International publication number: WO 2000/041707

(56) References cited:
- WO-A-98/35014
- MCFARLAND L V ET AL: "Pharmaceutical probiotics for the treatment of anaerobic and other infections." ANAEROBE, vol. 3, no. 2-3, 1997, pages 73-78, XP002137052 ISSN: 1075-9964
- CHARTERIS W P ET AL: "Development and application of an in vitro methodology to determine the transit tolerance of potentially probiotic Lactobacillus and Bifidobacterium species in the upper human gastrointestinal tract." JOURNAL OF APPLIED MICROBIOLOGY, vol. 84, no. 5, May 1998 (1998-05), pages 759-768, XP000929203 ISSN: 1364-5072

## Description

### Introduction

The invention relates to the use of strains of *Lactobacillus salivarius*.

The defence mechanisms to protect the human gastrointestinal tract from colonisation by intestinal bacteria are highly complex and involve both immunological and non-immunological aspects (V.J. McCracken and H.R. Gaskins, 'Probiotics a critical review', Horizon Scientific Press, UK, 1999, p. 278.). Innate defence mechanisms include the low pH of the stomach, bile salts, peristalsis, mucin layers and anti-microbial compounds such as lysozyme (D.C. Savage, 'Microbial Ecology of the Gut', Academic Press, London, 1997, p.278.). Immunological mechanisms include specialised lymphoid aggregates, underlying M cells, called peyers patches which are distributed throughout the small intestine and colon (M.F. Kagnoff. Gastroenterol. 1993, 105, 1275). Luminal antigens presented at these sites result in stimulation of appropriate T and B cell subsets with establishment of cytokine networks and secretion of antibodies into the gastrointestinal tract (M.R. Neutra and J-P Kraehenbuhl, 'Essentials of mucosal immunology', Academic Press, San Diego, 1996, p.29., M.E. Lamm. Ann. Rev. Microbiol. 1997, 51, 311). In addition, antigen presentation may occur via epithelial cells to intraepithelial lymphocytes and to the underlying lamina propria immune cells (S. Raychaudhuri et al. Nat Biotechnol., 1998, 16, 1025). Therefore, the host invests substantially in immunological defence of the gastrointestinal tract. However, as the gastrointestinal mucosa is the largest surface at which the host interacts with the external environment, specific control mechanisms must be in place to regulate immune responsiveness to the 100 tons of food which is handled by the gastrointestinal tract over an average lifetime (F. Shanahan, 'Physiology of the gastrointestinal tract', Raven Press, 1994, p.643.). Furthermore, the gut is colonised by over 500 species of bacteria numbering 10¹¹-10¹²/g in the colon. Thus, these control mechanisms must be capable of distinguishing non-pathogenic adherent bacteria from invasive pathogens which would cause significant damage to the host. In fact, the intestinal flora contributes to defence of the host by competing with newly ingested potentially pathogenic micro-organisms.

Consumption of non-pathogenic, or probiotic, bacteria has resulted in enhancement of immune parameters in healthy volunteers. Examples of these immune modulatory effects are given in Table 1.

**Table 1. Immune Enhancing Effects Following Oral Consumption of Probiotic Bacteria.**

| Observed Effect | Reference |
|---|---|
| Increased Macrophage Phagocytosis | 10 |
| Increased Natural Killer Cell Activity | 11 |
| Increased IFNγ serum levels | 12 |
| Increased B cell and NK cell numbers | 12 |
| Promotion of IgA responses | 11, 13-15 |
| Increased DTH responses | 16 |

Bacteria present in the human gastrointestinal tract can promote inflammation. Aberrant immune responses to the indigenous microflora have been implicated in certain disease states, such as inflammatory bowel disease (Brandzeag P. et al. Springer Semin. Immunopathol., 1997, 18, 555). Antigens associated with the normal flora usually lead to immunological tolerance and failure to achieve this tolerance is a major mechanism of mucosal inflammation (Stallmach A. et al., Immunol. Today, 1998, 19, 438). Evidence for this breakdown in tolerance includes an increase in antibody levels directed against the gut flora in patients with IBD.

WO-A-98/35014 describes strains of *Lactobacillus salivarius* isolated from resected and washed human gastrointestinal tract which inhibits a broad range of Gram positive and Gram negative microorganisms and which secretes a product having anti-microbial activity into a cell-free supernatant.

### Statements of Invention

The immune system is designed to defend host tissue and destroy invading pathogens. Upon recognition of the presence of a bacterial cell, cells of the immune system become activated and eliminate the bacterial threat. The production of inflammatory mediators promote cellular activation and pathogen destruction.

Surprisingly, we have found that strains of *Lactobacillus salivarius* elicit an anti-inflammatory effect *in vitro* and *in vivo.* We have found that the immune perception of *Lactobacillus salivarius* results in the suppression of inflammatory activity. The deliberate consumption in large numbers of *Lactobacillus salivarius* results in the suppression of inflammatory activity. The invention is therefore of major potential therapeutic value in the prophylaxis or treatment of undesirable inflammatory responses, such as inflammatory bowel disease.

*Lactobacillus salivarius* is a commensal microorganism originally isolated from the microbial flora within the human gastrointestinal tract. The immune system within the gastrointestinal tract cannot have a pronounced reaction to members of this flora as the resulting inflammatory activity would also destroy host cells and tissue function. Therefore, some mechanism(s) exist whereby the immune system can recognise commensal non-pathogenic members of the gastrointestinal flora as being different to pathogenic organisms. This ensures that damage to host tissues is restricted and a defensive barrier is still maintained.

According to the invention there is provided use of a strain of *Lactobacillus salivarius* of human origin in the preparation of a medicament for the prophylaxis and/or treatment of undesirable inflammatory activity.

The invention the undesirable inflammatory activity may be undesirable gastrointestinal inflammatory activity such as inflammatory bowel disease, eg. Crohns disease ulcerative colitis, irritable bowel syndrome, pouchitis or post infection colitis.

The inflammatory activity may be due to gastrointestinal cancer or systemic inflammatory disease such as rheumatoid arthritis.

In another instance the undesirable inflammatory activity may be due to an autoimmune disorder.

In yet another instance the undesirable inflammatory activity may be due to cancer.

In one embodiment the invention provides use of a strain of *Lactobacillus salivarius* in the preparation of a medicament for the prophylaxis of cancer.

In another embodiment the invention provides use of a strain of *Lactobacillus salivarius* wherein the *Lactobacillus salivarius* is contained in a formulation.

Preferably the formulation includes another probiotic material. Alternatively or additionally the formulation includes a prebiotic material.

Ideally the formulation includes an ingestable carrier. The ingestable carrier may be a pharmaceutically acceptable carrier such as a tablet, capsule or powder.

Preferably the ingestable carrier is a protein and/or a peptide, in particular proteins and/or peptides that are rich in glutamine/glutamate; a lipid; a carbohydrate; a vitamin; mineral and/or trace element.

Most preferably the ingestable carrier is a food product such as acidified milk, yoghurt, frozen yoghurt, milk powder, milk concentrate, cheese spreads, dressings or beverages.

In one embodiment the *Lactobacillus salivarius* is present in the formulation at more than 10⁶ cfu per gram of delivery system.

In another embodiment the formulation includes an adjuvant. The formulation may include a bacterial component. The formulation may alternatively or additionally include a drug entity. The formulation may also include a biological compound.

In one embodiment the invention provides use of a strain of *Lactobacillus salivarius* in the preparation of a medicament wherein the strain or formulation is for administration to animals. Preferably the animal is a mammal, most preferably a human.

In another embodiment the invention provides use of a strain of *Lactobacillus salivarius* wherein the *Lactobacillus salivarius* effects changes in an immunological marker when introduced into a system comprising cells which interact with the immune system and cells of the immune system.

Preferably the cells which interact with the immune system are epithelial cells. Most preferably the immunological marker is a cytokine especially TNFα.

Preferably the cells which interact with the immune system and the immune system cells are of matched origin.

In one embodiment the cells which interact with the immune system are of gastrointestinal, respiratory or genitourinary origin.

In another embodiment the cells of the immune system are of gastrointestinal, respiratory or genitourinary origin.

In a further embodiment the invention provides use of a strain of *Lactobacillus salivarius* wherein the *Lactobacillus salivarius* strain is *Lactobacillus salivarius* subspecies *salivarius.* Preferably the *Lactobacillus salivarius* is from resected and washed human gastrointestinal tract.

Preferably the *Lactobacillus salivarius* inhibits a broad range of Gram positive and Gram negative micro-organisms. Most preferably it secretes a product having anti-microbial activity into a cell-free supernatant, said activity being produced only by growing cells and being destroyed by proteinase K and pronase E.

An especially preferred strain of *Lactobacillus salivarius* is *Lactobacillus salivarius* strain UCC 188 or mutant or variant thereof.

A deposit of *Lactobacillus salivarius* strain UCC 118 was made at the NCIMB on November 27, 1996 and accorded the accession number NCIMB 40829. The strain of. *Lactobacillus salivarius* is described in WO-A-98/35014.

The *Lactobacillus salivarius* may be a genetically modified mutant or it may be a naturally occurring variant of *Lactobacillus salivarius*.

Preferably the *Lactobacillus salivarius* is in the form of viable cells. Alternatively the *Lactobacillus salivarius* may be in the form of non-viable cells.

### Brief description of the drawings

Fig. 1 is a graph of *C. perfringens* levels in the mice consuming UCC118 compared to a placebo group (p<0.05). Results are plotted as the mean log values ± standard error for each of the groups
Fig. 2 is a bar chart of inflammatory scores for mice consuming UCC118 in comparison to control mice. Results are shown as the mean ± standard error for each of the groups.
Fig. 3 is a graph of TNFα levels over six weeks that patients consume UCC118. Results are plotted as the mean pg/ml TNFα level for each time point (n=22).
Fig. 4 is a graph of CDAI scores for patients consuming UCC 118 over the course of probiotic feeding. CDAI scores decreased from an average of 180 to 160.
Fig. 5 is a graph of cytokine production *in vitro* following exposure to UCC118, Results are expressed as pg/ml.
Fig. 6 is bar chart of extracellular TNFα, IL-1RA, IL-6, sIL-6R, and IFNα levels following exposure to *Lactobacillus salivarius* UCC118.
Fig. 7 is a gene array with specific gene sequences for 268 cytokines and related molecules to examine the immune response to UCC118. The bottom panel illustrates the control culture while the top panel illustrates cytokine gene expression by PBMCs following exposure to UCC118; and
Fig. 8 is a bar chart of TNFα levels in the presence of various bacterial strains.

### Detailed Description

We have developed criteria for *in vitro* selection of probiotic bacteria that reflect certain *in vivo* effects on their host, such as modulation of the GIT microflora and modulation of the mucosal immune response resulting in the production of secretory antibodies specific to the consumed strain. We have found that *Lactobacillus salivarius* subsp. *salivarius* UCC118 not only survives passage through the gastrointestinal tract and adheres to human intestinal cell lines but also, surprisingly has anti-inflammatory effects.

The general use of probiotic bacteria is in the form of viable cells. However, it can also be extended to non-viable cells such as killed cultures or compositions containing beneficial factors expressed by the probiotic bacteria. This could include thermally killed micro-organisms or micro-organisms killed by exposure to altered pH or subjection to pressure. With non-viable cells product preparation is simpler, cells may be incorporated easily into pharmaceuticals and storage requirements are much less limited than viable cells. *Lactobacillus casei* YIT 9018 offers an example of the effective use of heat killed cells as a method for the treatment and/or prevention of tumour growth as described in US Patent No. US4347240.

It is unknown whether intact bacteria are required to exert an anti-inflammatory effect or if individual active components of the invention can be utilised alone. Proinflammatory components of certain bacterial strains have been identified. The proinflammatory effects of gram-negative bacteria are mediated by lipopolysaccharide (LPS). LPS alone induces a proinflammatory network, partially due to LPS binding to the CD14 receptor on monocytes. It is assumed that components of probiotic bacteria possess anti-inflammatory activity, due to the effects of the whole cell. Upon isolation of these components, pharmaceutical grade manipulation is anticipated.

The invention will be more clearly understood from the following examples.

### Example 1: Detailed description of the in vivo demonstration of the anti-inflammatory effects of Lactobacillus salivarius especially subspecies salivarius UCC118.

### Murine model of gastrointestinal inflammation

Aberrant immune responses to the indigenous microflora have been implicated in certain disease states, such as inflammatory bowel disease (Brandzeag P., et al. Springer Semin. Immunopathol., 1997, 18, 555). Antigens associated with the normal flora usually lead to immunological tolerance and failure to achieve this tolerance is a major mechanism of mucosal inflammation (Stallmach A., et al. Immunol. Today, 1998, 19, 438). Evidence for this breakdown in tolerance includes an increase in antibody levels directed against the gut flora in patients with IBD. In addition, certain mouse models predisposed to inflammatory lesions in the gastrointestinal tract remain disease free when housed in germ free conditions or when treated with antibiotics (Kuhn R., et al. Cell, 1993, 75, 263; Panwala C. M., et al. J. Immunol., 1998, 161, 5733).

C57BL/6 Interleukin-10 knockout mice are predisposed to developing enterocolitis in the presence of an enteric bacterial flora. When maintained in germ free conditions, IL-10 knock out mice do not develop the disease (Kuhn R., et al. Cell, 1993, 75, 263). Since the pathogenesis of this disease has been linked with the enteric flora, elimination of specific components of this flora may have a beneficial effect on the severity of this disease.

*Lactobacillus salivarius* subsp. *salivarius* UCC118 is a probiotic bacteria, which was isolated from a healthy human ileum. It is suited to gastrointestinal colonization as it fulfills many criteria set down for the selection of probiotic strains. These include traits such as bile tolerance, acid resistance and *in vitro* adherence to human colonic cell lines. Feeding trials in healthy humans have been conducted and considerable modification of the gastrointestinal flora was noted. In addition, UCC118 was perceived by the mucosal immune system resulting in the production and secretion of IgA specific to UCC118.

Thus, UCC118 survives passage through the gastrointestinal tract, modulates the gut flora and is perceived by the mucosal immune system. The influence of this probiotic bacteria in modulating inflammatory responses within the gastrointestinal tract was examined using a murine model of enterocolitis. In addition, we examined the role of *Lactobacillus salivarius* subsp. *salivarius* UCC118 in reducing the rate of neoplastic change within the gastrointestinal tract.

Twenty IL-10 KO mice were studied (ten consumed probiotic organisms in milk and 10 consumed unmodified milk) for 16 weeks. Fecal microbial analysis was performed weekly to enumerate excretion of lactobacilli, *Clostridium perfringens,* bacteroides, coliforms, bifidobacteria and enterococci. At sacrifice, small and large bowel were microbiologically and histologically assessed.

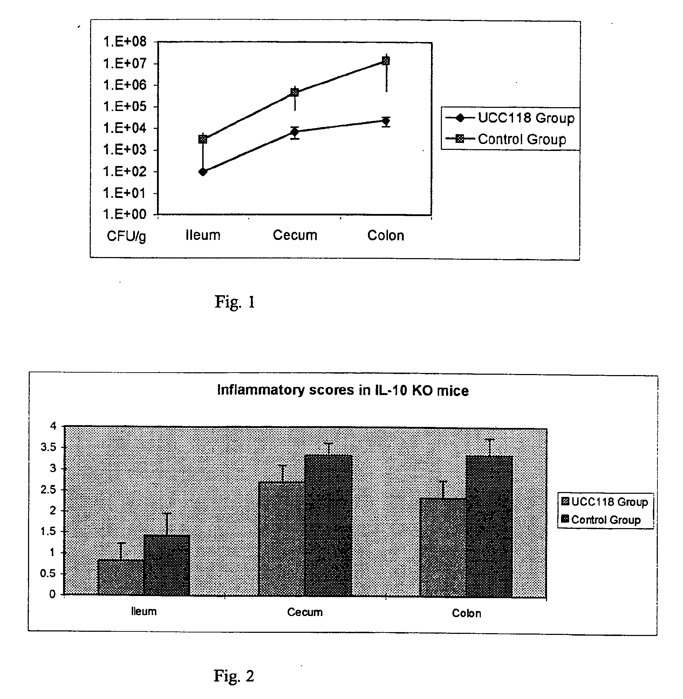

Fecal coliform and enterococci levels were significantly reduced in test animals compared to the controls. At sacrifice, a significant reduction in *C. perfringens* numbers was observed in the test mice (Figure 1). There were no fatalities in the test group compared to two deaths from fulminant colitis in the control group. Only one test mouse developed colonic adenocarcinoma compared to five in the control group. Test animal mucosal inflammation consistently scored lower than that of the control mice (Figure 2). The reduction in tumour incidence following consumption of UCC118 may be related to the reduced level of inflammation within the gastrointestinal tract or may be due to elimination of pro-carcinogenic members of the gastrointestinal flora (Rumney C.J., et al. Carcinogenesis, 1993, 14, 79; Rowland I.R. (1995). In: Gibson G.R. (ed). Human colonic bacteria: role in nutrition, physiology and pathology, pp 155-174. Boca Raton CRC Press; Darveau D. Nat. Biotech., 1999, 17, 19).

In conclusion, consumption of *Lactobacillus salivarius* UCC 118 results in a significant modulation of the gut flora and an improvement in mortality rate, cancer incidence and disease score.

### Example 2: Human Trial with UCC118 in patients with active Crohn's disease.

Inflammatory bowel disease (IBD) encompasses a number of inflammatory disorders of the gastrointestinal tract, including both Crohn's disease and ulcerative colitis.

Patients suffering from active Crohn's disease have been treated with UCC118. Briefly, UCC118 was consumed in a fermented milk product for 6 weeks by 22 patients. Microbiological and immunological determinations were made at week 0, week 1, week 3 and week 6. This was not a placebo-controlled trial.

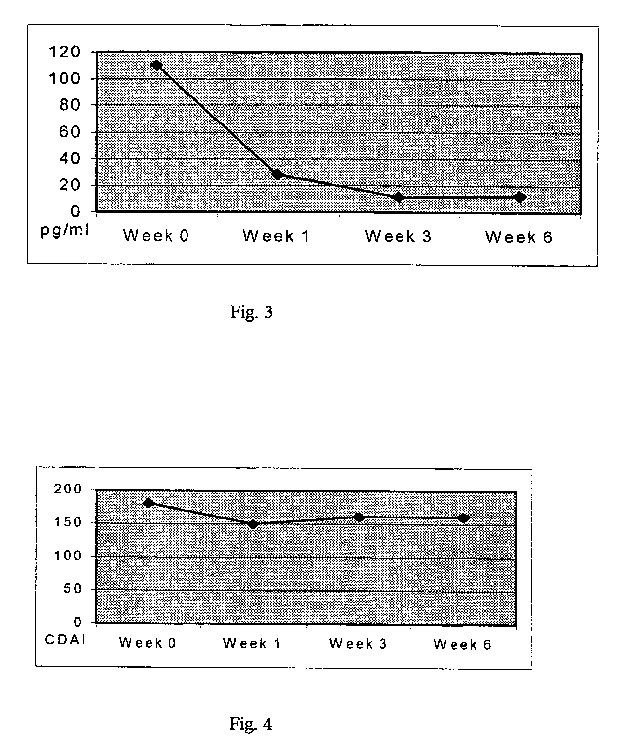

A number of systemic cytokine levels were measured over the course of feeding. In particular, tumour necrosis factor α (TNFα), a proinflammatory cytokine that has been implicated in the pathogenesis of many inflammatory disease states, including inflammatory bowel disease. Current therapies for inflammatory bowel disease specifically aim to reduce TNFα levels (Present D.H., et al. New Eng. J. Med., 1999, 340, 1398). In this trial, systemic TNFα levels were reduced following consumption of UCC118 (Figure 3).

In addition, patients were assessed regarding their Crohn's Disease Activity Index (CDAI) over the six week trial period. This index assesses the general health and well being of each patient (Figure 4). Overall, the disease activity index improved slightly for the majority of individuals in the trial. These are patients with moderately active disease and their CDAI scores would be expected to increase. However, following treatment with UCC118, CDAI scores did not increase and in fact they improved from a mean of 180 to 160.

### Example 3: Detailed description of the in vitro demonstration of the mechanisms underlying the anti-inflammatory effects of Lactobacillus salivarius especially subspecies salivarius UCC118.

A number of methodologies have been utilised for these studies including ELISAs (extracellular protein determination), flow cytometry (intracellular protein determination) and cDNA expression arrays (mRNA expression). In particular, examination of the expression of tumour necrosis factor α has been targeted, due to its clinical importance, and suppression of the production of this cytokine, following exposure to UCC118, has been noted using all three methodologies.

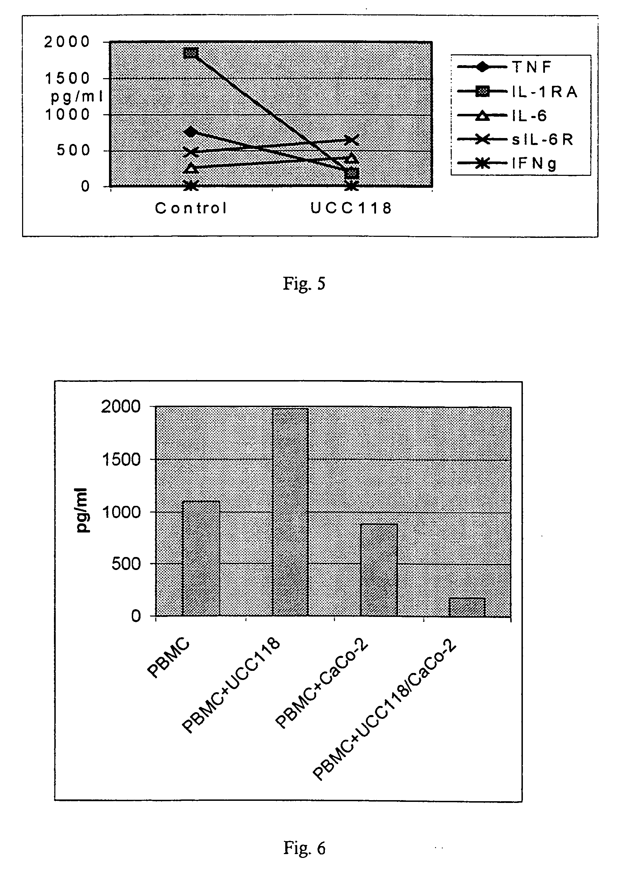

Using a transwell assay system, with epithelial cells and peripheral blood mononuclear cells, extracellular cytokine levels were measured by ELISAs. Following co-incubation with UCC118, the amount of TNFα produced was significantly reduced compared to control cultures. Furthermore, IL-1RA and IFNγ levels dropped while IL-6 and soluble IL-6 receptor levels increased (Figure 5). Intracellular staining for TNFα confirmed the ELISA result as TNFα levels were lower in the UCC118 stimulated sample compared to controls.

Figure 6 demonstrates the tricellular signalling that occurs. Co-incubation of PBMCs and *Lactobacillus salivarius* strain UCC118 results in the stimulation of TNFα production. However, co-incubation of PBMCs, *Lactobacillus salivarius* strain UCC118 and epithelial cells (CaCo-2 cells) results in a significant inhibition of TNFα production. Thus, a significantly different pattern of signalling is present in the tricellular model compared to bacteria and PBMCs alone.

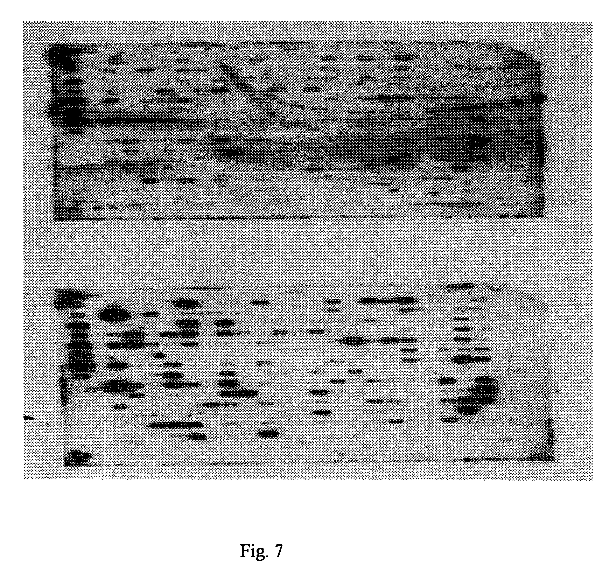

Gene arrays measure the quantity of mRNA in a population of cells. We stimulated peripheral blood mononuclear cells with UCC118 for 24 hours and we examined the effect on cytokine gene expression (Figure 7). Considerable modification of cytokine gene expression was noted. For example, genes encoding the proinflammatory cytokines IL-1β and TNFα were turned off while genes encoding Th2 type cytokines, such as IL-6, were enhanced.

*In vitro* models have demonstrated that UCC118 is capable of inducing Th2 type cytokines (i.e. IL-6 and IL-6 soluble receptor) while suppressing the production of inflammatory cytokines such as TNFα and IL-1β. Thus, these results suggest that consumption of UCC118 would be of benefit to patients suffering from inflammatory diseases, such as IBD.

### Example 4: Test for anti-inflammatory bacterial strains

A number of lactic acid bacteria, which have been isolated from the human gastrointestinal tract, were examined in this novel assay system for anti-inflammatory effect. All bacterial strains were taken from -20°C glycerol stocks and incubated anaerobically overnight in MRS broth and washed in antibiotic containing medium. Epithelial cell monolayers were grown for 6 weeks prior to the addition of PBMCs and bacterial cells.

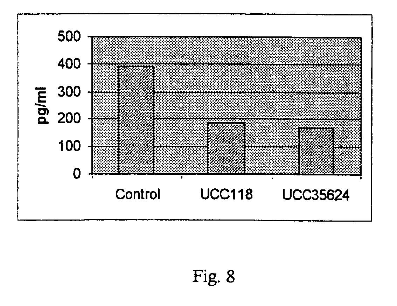

The results of these stimulations can be observed in Figure 8. Relative to control cultures, two bacterial strains suppressed TNFα production. The two strains *Lactobacillus salivarius* strain UCC118, which suppressed production of TNFα, is the subject of WO-A-9835014. The *Bifidobacterium longum infantis* strain UCC 35624 is the subject of a PCT Application filed concurrently with the present application.

### Inflammation

Inflammation is the term used to describe the local accumulation of fluid, plasma proteins and white blood cells at a site that has sustained physical damage, infection or where there is an ongoing immune response. Control of the inflammatory response is exerted on a number of levels (for review see Henderson B., and Wilson M. 1998. In "Bacteria-Cytokine interactions in health and disease. Portland Press, 79-130). The controlling factors include cytokines, hormones (e.g. hydrocortisone), prostaglandins, reactive intermediates and leukotrienes. Cytokines are low molecular weight biologically active proteins that are involved in the generation and control of immunological and inflammatory responses, while also regulating development, tissue repair and haematopoiesis. They provide a means of communication between leukocytes themselves and also with other cell types. Most cytokines are pleiotrophic and express multiple biologically overlapping activities. Cytokine cascades and networks control the inflammatory response rather than the action of a particular cytokine on a particular cell type (Arai KI, et al., Annu Rev Biochem 1990;59:783-836). Waning of the inflammatory response results in lower concentrations of the appropriate activating signals and other inflammatory mediators leading to the cessation of the inflammatory response. TNFαis a pivotal proinflammatory cytokine as it initiates a cascade of cytokines and biological effects resulting in the inflammatory state. Therefore, agents which inhibit TNFαare currently being used for the treatment of inflammatory diseases, e.g. Infliximab (Trade Mark).

Pro-inflammatory cytokines are thought to play a major role in the pathogenesis of many inflammatory diseases, including inflammatory bowel disease (IBD). Current therapies for treating IBI) are aimed at reducing the levels of these pro-inflammatory cytokines, including IL-8 and TNFα Such therapies may also play a significant role in the treatment of systemic inflammatory diseases such as rheumatoid arthritis.

In view of the anti-inflammatory properties of *Lactobacillus salivarius* that we have discovered these strains may have potential application in the treatment of a range of inflammatory diseases, particularly if used in combination with other anti-inflammatory therapies, such as non-steroid anti-inflammatory drugs (NSAIDs) or Infliximab (Trade Mark).

### Autoimmune Disease

The immune system has a large repertoire of specificities expressed by B and T cells. Some of these specificities will be directed to self-components. Self-recognition is normally controlled by clonal deletion and inactivation of self-reactive lymphocytes. However, there is a constant background of autoimmunity with antibodies to many proteins being found in serum. A breakdown in the self-nonself recognition system results in autoimmunity. When autoimmune disease does occur, the resulting immune response damages the tissue bearing the offending antigen. Immune complex deposition, type II hypersensitivity and cell-mediated reactions are the most important mechanisms by which immunopathological damage occurs. Examples of autoimmune diseases include, but are not limited to, systemic lupus erythematosus, rheumatoid arthritis, insulin dependent diabetes mellitus, myasthenia gravis and pernicious anaemia. We have found that *Lactobacillus salivarius* is an immunomodulatory bacterium. Thus, consumption either as a single component or in combination with other bacteria by patients suffering from autoimmune disease may restrict organ damage and help restore normal body homeostasis.

### Inflammation and Cancer

The production of multifunctional cytokines across a wide spectrum of tumour types suggests that significant inflammatory responses are ongoing in patients with cancer. It is currently unclear what protective effect this response has against the growth and development of tumour cells *in vivo.* However, these inflammatory responses could adversely affect the tumour bearing host. Complex cytokine interactions are involved in the regulation of cytokine production and cell proliferation within tumour and normal tissues (McGee DW, et al., Immunology 1995 Sep;86(1):6-11, Wu S, et al., Gynecol Oncol 1994 Apr;53(1):59-63). It has long been recognised that weight loss (cachexia) is the single most common cause of death in patients with cancer (Inagaki J, et al., Cancer 1974 Feb;33(2):568-73) and initial malnutrition indicates a poor prognosis (Van Eys J. Nutr Rev 1982 Dec;40(12):353-9). For a tumour to grow and spread it must induce the formation of new blood vessels and degrade the extracellular matrix. The inflammatory response may have significant roles to play in the above mechanisms, thus contributing to the decline of the host and progression of the tumour. Due to the anti-inflammatory properties of *Lactobacillus salivarius* these bacterial strains they may reduce the rate of malignant cell transformation. Furthermore, intestinal bacteria can produce, from dietary compounds, substances with genotoxic, carcinogenic and tumour-promoting activity and gut bacteria can activate pro-carcinogens to DNA reactive agents (Rowland I.R. (1995). Toxicology of the colon: role of the intestinal microflora. In: Gibson G.R. (ed). Human colonic bacteria: role in nutrition, physiology and pathology, pp 155-174. Boca Raton CRC Press). In general, species of *Lactobacillus* have low activities of xenobiotic metabolising enzymes compared to other populations within the gut such as bacteroides, eubacteria and clostridia (Saito Y., et al., Microb. Ecol. Health Dis., 1992;5, 105-110). Therefore, increasing the number of *Lactobacillus* bacteria in the gut could beneficially modify the levels of these enzymes.

### Prebiotics

The introduction of probiotic organisms is accomplished by the ingestion of the microorganism in a suitable carrier. It would be advantageous to provide a medium that would promote the growth of these probiotic strains in the large bowel. The addition of one or more oligosaccharides, polysaccharides, or other prebiotics enhances the growth of lactic acid bacteria in the gastrointestinal tract (Gibson, GR. Br. J. Nutr. 1998;80 (4):S209-12). Prebiotics refers to any non-viable food component that is specifically fermented in the colon by indigenous bacteria thought to be of positive value, e.g. bifidobacteria, lactobacilli. Types of prebiotics may include those which contain fructose, xylose, soya, galactose, glucose and mannose. The combined administration of a probiotic strain with one or more prebiotic compounds may enhance the growth of the administered probiotic *in vivo* resulting in a more pronounced health benefit, and is termed synbiotic.

### Other active ingredients

It will be appreciated that the *Lactobacillus salivarius* may be administered prophylactically or as a method of treatment either on its own or with other probiotic and/or prebiotic materials as described above. In addition, the bacteria may be used as part of a prophylactic or treatment regime using other active materials such as those used for treating inflammation or other disorders, especially those of the gastrointestinal tract. Such combinations may be administered in a single formulation or as separate formulations administered at the same or different times and using the same or different routes of administration.

The invention is not limited to the embodiments hereinbefore described which may be varied in detail.

## Claims

1. Use of a strain of *Lactobacillus salivarius* of human origin in the preparation of a medicament for the prophylaxis and/or treatment of undesirable inflammatory activity.

2. Use as claimed in claim 1 wherein the undesirable inflammatory activity is undesirable gastrointestinal inflammatory activity.

3. Use as claimed in claim 2 wherein the gastrointestinal inflammatory activity is inflammatory bowel disease.

4. Use as claimed in claim 3 wherein the gastrointestinal inflammatory activity is Crohns disease.

5. Use as claimed in claim 3 wherein the gastrointestinal activity is ulcerative colitis.

6. Use as claimed in claim 2 wherein the gastrointestinal inflammatory activity is irritable bowel syndrome.

7. Use as claimed in claim 2 wherein the gastrointestinal inflammatory activity is pouchitis.

8. Use as claimed in claim 2 wherein the gastrointestinal inflammatory activity is post infection colitis.

9. Use as claimed in claim 2 wherein the inflammatory activity is due to gastrointestinal cancer.

10. Use as claimed in claim 1 wherein the inflammatory activity is systemic inflammatory disease.

11. Use as claimed in claim 10 wherein the systemic inflammatory disease is rheumatoid arthritis.

12. Use as claimed in claim 1 or 2 wherein the undesirable inflammatory activity is due to an autoimmune disorder.

13. Use as claimed in claim wherein the undesirable inflammatory activity is due to cancer.

14. Use of a strain of *Lactobacillus salivarius* in the preparation of a medicament for the prophylaxis of cancer.

15. Use as claimed in any of claims 1 to 14 wherein the *Lactobacillus salivarius* is contained in a formulation.

16. Use as claimed in claim 15 wherein the formulation includes another probiotic material.

17. Use as claimed in claim 15 or 16 wherein the formulation includes a prebiotic material,

18. Use as claimed in any of claims 15 to 17 wherein the formulation includes an ingestable currier, preferably the ingestable carrier is a pharmaceutically acceptable carrier such as a tablet, capsule or powder.

19. Use as claimed in any of claims 15 to 18 wherein the ingestabte carrier is a protein and/or a peptide, in particular proteins and/or peptides that are rich in glutamine/glutamate; a lipid; a carbohydrate; a vitamin; mineral and/or trace element.

20. Use as claimed in any of claims 15 to 19 wherein the ingestable carrier is a food product such as acidified milk, yoghurt, frozen yoghurt, milk powder, milk concentrate, cheese spreads, dressings or beverages.

21. Use as claimed in any of claims 15 to 20 wherein the *Lactobacillus salivarius* is present at more than 10⁶ cfu per gram of delivery system.

22. Use as claimed in any or claims 15 to 21 wherein the formulation includes any one or more of an adjuvant, bacterial components, a drug entity, or a biological compound.

23. Use as claimed in any preceding claim wherein the strain or formulation is for administration to animals.

24. Use as claimed in claim 23 wherein the animal is a mammal, preferably a human.

25. Use as claimed in any preceding claim wherein the *Lactobacillus salivarius,* effects changes in an immunological marker when introduced into a system comprising cells which interact with the immune system and cells of the immune system.

26. Use- as claimed in claim 25 wherein the cells which interact with the immune system are epithelial cells.

27. Use as claimed in claim 25 or 26 wherein the immunological marker is a cytokine, preferably the cytokine is TNFα.

28. Use as claimed in any of claims 25 to 27 wherein the cells which interact with the immune system and the immune system cells are of matched origin.

29. Use as claimed in any of claims 25 to 28 wherein the cells which interact with the immune system are of gastrointestinal, respiratory or genitourinary origin.

30. Use as claimed in any of claims 25 to 29 wherein the cells of the immune system are of gastrointestinal, respiratory or genitourinary origin.

31. Use as claimed in any preceding claim wherein the *Lactobacillus salivarius* strain is *Lactobacillus salivarius* subspecies *salivarius.*

32. Use as claimed in any preceding claim wherein the *Lactobacillus salivarius* is isolated from resected and washed human gastrointestinal tract.

33. Use as claimed in any preceding claim wherein the *Lactobacillus salivarius* inhibits a broad range of Gram positive and Gram negative micro-organisms.

34. Use as claimed in any preceding claim wherein *Lactobacillus salivarius* secretes a product having anti-microbial activity into a cell-free supernatant, said activity being produced only by growing cells and being destroyed by proteinase K and pronase E.

35. Use as claimed in any preceding claim wherein the *Lactobacillus salivarius* strain is strain UCC 118 [NCIMB40829] or a mutant or variant thereof.

36. Use as claimed in any preceding claim wherein the *Lactobacillus salivarius* is a genetically modified mutant.

37. Use as claimed in any preceding claim wherein the *Lactobacillus salivarius* is a naturally occurring variant of *Lactobacillus salivarius.*

38. Use as claimed in any preceding claim wherein the *Lactobacillus salivarius* is in the form of viable cells.

39. Use as claimed in any preceding claims wherein the *Lactobacillus salivarius* is in the form of non-viable cells.

## Patentansprüche

1. Verwendung eines Stammes von *Lactobacillus salivarius* von humaner Herkunft bei der Herstellung eines Arzneimittels für die Prophylaxe und/oder Behandlung von unerwünschter entzündlicher Aktivität.

2. Verwendung nach Anspruch 1, worin die unerwünschte entzündliche Aktivität eine unerwünschte gastrointestinale entzündliche Aktivität darstellt.

3. Verwendung nach Anspruch 2, worin die gastrointestinale entzündliche Aktivität eine entzündliche Darmerkrankung darstellt.

4. Verwendung nach Anspruch 3, worin die gastrointestinale entzündliche Aktivität die Crohn-Krankheit darstellt.

5. Verwendung nach Anspruch 3, worin die gastrointestinale entzündliche Aktivität ulcerative Colitis darstellt.

6. Verwendung nach Anspruch 2, worin die gastrointestinale entzündliche Aktivität das Reizdarmsyndrom darstellt.

7. Verwendung nach Anspruch 2, worin die gastrointestinale entzündliche Aktivität Pouchitis darstellt.

8. Verwendung nach Anspruch 2, worin die gastrointestinale entzündliche Aktivität eine postinfektiöse Colitis darstellt.

9. Verwendung nach Anspruch 2, worin die entzündliche Aktivität auf gastrointestinalen Krebs zurückzuführen ist.

10. Verwendung nach Anspruch 1, worin die entzündliche Aktivität eine systemische entzündliche Erkrankung darstellt.

11. Verwendung nach Anspruch 10, worin die systemische entzündliche Erkrankung rheumatoide Arthritis darstellt.

12. Verwendung nach Anspruch 1 oder 2, worin die unerwünschte entzündliche Aktivität auf eine Autoimmunerkrankung zurückzuführen ist.

13. Verwendung nach Anspruch 1, worin die unerwünschte entzündliche Aktivität auf Krebs zurückzuführen ist.

14. Verwendung eines Stammes von *Lactobacillus salivarius* bei der Herstellung eines Arzneimittels für die Krebs-Prophylaxe.

15. Verwendung nach einem der Ansprüche 1 bis 14, worin der *Lactobacillus salivarius* in einer Formulierung enthalten ist.

16. Verwendung nach Anspruch 15, worin die Formulierung ein anderes probiotisches Material einschließt.

17. Verwendung nach Anspruch 15 oder 16, worin die Formulierung ein präbiotisches Material einschließt.

18. Verwendung nach einem der Ansprüche 15 bis 17, worin die Formulierung einen aufnehmbaren Träger einschließt, wobei der aufnehmbare Träger bevorzugt einen pharmazeutisch verträglichen Träger, wie zum Beispiel eine Tablette, eine Kapsel oder ein Pulver, darstellt.

19. Verwendung nach einem der Ansprüche 15 bis 18, worin der aufnehmbare Träger ein Protein und/oder ein Peptid, insbesondere Proteine und/oder Peptide darstellt, die reich an Glutamin/Glutamat; einem Lipid, einem Kohlenhydrat; einem Vitamin; einem Mineral und/oder einem Spurenelement sind.

20. Verwendung nach einem der Ansprüche 15 bis 19, worin der aufnehmbare Träger ein Lebensmittelprodukt, wie zum Beispiel gesäuerte Milch, Joghurt, gefrorener Joghurt, Milchpulver, Milchkonzentrat, Streichkäse, Dressings oder Getränke darstellt.

21. Verwendung nach einem der Ansprüche 15 bis 20, worin der *Lactobacillus salivarius* bei mehr als 10⁶ KBE pro Gramm bezogen auf das Abgabesystem anwesend ist.

22. Verwendung nach einem der Ansprüche 15 bis 21, worin die Formulierung jedwede(s) eine oder mehr von einem Adjuvans, bakteriellen Komponenten, einer Arzneimittelentität oder einer biologischen Verbindung einschließt.

23. Verwendung nach einem der vorangehenden Ansprüche, worin der Stamm oder die Formulierung zur Verabreichung an Tiere bestimmt ist.

24. Verwendung nach Anspruch 23, worin das Tier einen Säuger, bevorzugt einen Menschen darstellt.

25. Verwendung nach einem der vorangehenden Ansprüche, worin der *Lactobacillus salivarius* Veränderungen in einem immunologischen Marker bewirkt, wenn er in ein System eingeführt wird, umfassend Zellen, die mit dem Immunsystem interagieren, und Zellen des Immunsystems.

26. Verwendung nach Anspruch 25, worin die Zellen, die mit dem Immunsystem interagieren, Epithelzellen darstellen.

27. Verwendung nach Anspruch 25 oder 26, worin der immunologische Marker ein Cytokin darstellt, wobei das Cytokin bevorzugt TNFα darstellt.

28. Verwendung nach einem der Ansprüche 25 bis 27, worin die Zellen, die mit dem Immunsystem interagieren, und die Zellen des Immunsystems von übereinstimmender Herkunft sind.

29. Verwendung nach einem der Ansprüche 25 bis 28, worin die Zellen, die mit dem Immunsystem interagieren, von gastrointestinaler, respiratorischer oder genitourinärer Herkunft sind.

30. Verwendung nach einem der Ansprüche 25 bis 29, worin die Zellen des Immunsystems von gastrointestinaler, respiratorischer oder genitourinärer Herkunft sind.

31. Verwendung nach einem der vorangehenden Ansprüche, worin der *Lactobacillus salivarius*-Stamm *Lactobacillus salivarius* subsp. *salivarius* darstellt.

32. Verwendung nach einem der vorangehenden Ansprüche, worin der *Lactobacillus salivarius* aus dem resezierten und gewaschenen humanen Gastrointestinaltrakt isoliert wird.

33. Verwendung nach einem der vorangehenden Ansprüche, worin der *Lactobacillus salivarius* ein breites Spektrum Gram-positiver und Gram-negativer Mikroorganismen inhibiert.

34. Verwendung nach einem der vorangehenden Ansprüche worin der *Lactobacillus salivarius* ein Produkt mit antimikrobieller Aktivität in einen zellfreien Überstand sezerniert, wobei die genannte Aktivität nur von wachsenden Zellen produziert und von Proteinase K und Pronase E zerstört wird.

35. Verwendung nach einem der vorangehenden Ansprüche, worin der Stamm von *Lactobacillus salivarius* den Stamm UCC 118 [NCIMB 40829] oder eine Mutante oder Variante davon darstellt.

36. Verwendung nach einem der vorangehenden Ansprüche, worin der *Lactobacillus salivarius* eine genetisch modifizierte Mutante darstellt.

37. Verwendung nach einem der vorangehenden Ansprüche, worin der *Lactobacillus salivarius* eine natürlich vorkommende Variante von *Lactobacillus salivarius* darstellt.

38. Verwendung nach einem der vorangehenden Ansprüche, worin der *Lactobacillus salivarius* in der Form von lebensfähigen Zellen vorliegt.

39. Verwendung nach einem der vorangehenden Ansprüche, worin der *Lactobacillus salivarius* in der Form von nicht lebensfähigen Zellen vorliegt.

## Revendications

1. Utilisation d'une souche de *Lactobacillus salivarius* d'origine humaine dans la préparation d'un médicament destiné à la prophylaxie et/ou au traitement d'une activité inflammatoire indésirable.

2. Utilisation selon la revendication 1 dans laquelle l'activité inflammatoire indésirable est une activité inflammatoire gastro-intestinale indésirable.

3. Utilisation selon la revendication 2 dans laquelle l'activité inflammatoire gastro-intestinale est une maladie intestinale inflammatoire.

4. Utilisation selon la revendication 3 dans laquelle l'activité inflammatoire gastro-intestinale est la maladie de Crohn.

5. Utilisation selon la revendication 3 dans laquelle l'activité gastro-intestinale est une colite ulcéreuse.

6. Utilisation selon la revendication 2 dans laquelle l'activité inflammatoire gastro-intestinale est le syndrome de l'intestin irritable.

7. Utilisation selon la revendication 2 dans laquelle l'activité inflammatoire gastro-intestinale est une pouchite.

8. Utilisation selon la revendication 2 dans laquelle l'activité inflammatoire gastro-intestinale est une colite post-infection.

9. Utilisation selon la revendication 2 dans laquelle l'activité inflammatoire est due à un cancer gastro-intestinal.

10. Utilisation selon la revendication 1 dans laquelle l'activité inflammatoire est une maladie inflammatoire systémique.

11. Utilisation selon la revendication 10 dans laquelle la maladie inflammatoire systémique est une polyarthrite rhumatoïde.

12. Utilisation selon la revendication 1 ou 2 dans laquelle l'activité inflammatoire indésirable est due à un trouble auto-immun.

13. Utilisation selon la revendication 1 dans laquelle l'activité inflammatoire indésirable est due à un cancer.

14. Utilisation d'une souche de *Lactobacillus salivarius* dans la préparation d'un médicament destiné à la prophylaxie d'un cancer.

15. Utilisation selon l'une quelconque des revendications 1 à 14 dans laquelle le *Lactobacillus salivarius* est renfermé dans une formulation.

16. Utilisation selon la revendication 15 dans laquelle la formulation inclut une autre substance probiotique.

17. Utilisation selon la revendication 15 ou 16 dans laquelle la formulation inclut une substance prébiotique.

18. Utilisation selon l'une quelconque des revendications 15 à 17 dans laquelle la formulation inclut une véhicule ingérable, le véhicule ingérable étant de préférence un véhicule pharmaceutiquement acceptable tel qu'un comprimé, une capsule ou une poudre.

19. Utilisation selon l'une quelconque des revendications 15 à 18 dans laquelle le véhicule ingérable est une protéine et/ou un peptide, en particulier des protéines et/ou des peptides qui sont riches en glutamine et glutamate ; un lipide ; un glucide ; une vitamine ; un minéral et/ou un oligo-élément.

20. Utilisation selon l'une quelconque des revendications 15 à 19 dans laquelle le véhicule ingérable est un produit alimentaire tel que du lait acidifié, du yaourt, du yaourt glacé, du lait en poudre, du lait concentré, des fromages à tartiner, des sauces ou des boissons.

21. Utilisation selon l'une quelconque des revendications 15 à 20 dans laquelle le *Lactobacillus salivarius* est présent à plus de 10⁶ cfu par gramme de système d'administration.

22. Utilisation selon l'une quelconque des revendications 15 à 21 dans laquelle la formulation inclut l'un quelconque ou plusieurs des constituants parmi un adjuvant, des composants bactériens, une entité médicamenteuse ou un composé biologique.

23. Utilisation selon une quelconque revendication précédente dans laquelle la souche ou la formulation est destinée à être administrée à des animaux.

24. Utilisation selon la revendication 23 dans laquelle l'animal est un mammifère, de préférence un humain.

25. Utilisation selon une quelconque revendication précédente dans laquelle le *Lactobacillus salivarius* effectue des changements dans un marqueur immunologique lorsqu'il est introduit dans un système comprenant des cellules qui interagissent avec le système immunitaire et des cellules du système immunitaire.

26. Utilisation selon la revendication 25 dans laquelle les cellules qui interagissent avec le système immunitaire sont des cellules épithéliales.

27. Utilisation selon la revendication 25 ou 26 dans laquelle le marqueur immunologique est une cytokine, la cytokine étant de préférence la TNFα.

28. Utilisation selon l'une quelconque des revendications 25 à 27 dans laquelle les cellules qui interagissent avec le système immunitaire et les cellules du système immunitaire sont d'origine appariée.

29. Utilisation selon l'une quelconque des revendications 25 à 28 dans laquelle les cellules qui interagissent avec le système immunitaire sont d'origine gastro-intestinale, respiratoire ou génito-urinaire.

30. Utilisation selon l'une quelconque des revendications 25 à 29 dans laquelle les cellules du système immunitaire sont d'origine gastro-intestinale, respiratoire ou génito-urinaire.

31. Utilisation selon une quelconque revendication précédente dans laquelle la souche de *Lactobacillus salivarius* est le *Lactobacillus salivarius* de la sous-espèce *salivarius.*

32. Utilisation selon une quelconque revendication précédente dans laquelle le *Lactobacillus salivarius* est isolé à partir d'un appareil digestif humain réséqué et lavé.

33. Utilisation selon une quelconque revendication précédente dans laquelle le *Lactobacillus salivarius* inhibe une grande plage de micro-organismes Gram positifs et Gram négatifs.

34. Utilisation selon une quelconque revendication précédente dans laquelle le *Lactobacillus salivarius* secrète un produit qui a une activité anti-microbienne dans un surnageant sans cellules, ladite activité étant produite seulement par des cellules en croissance et étant supprimée par la protéinase K et la pronase E.

35. Utilisation selon une quelconque revendication précédente dans laquelle la souche de *Lactobacillus salivarius* est la souche UCC 118 [NCIMB40829] ou un mutant ou une variante de celle-ci.

36. Utilisation selon une quelconque revendication précédente dans laquelle le *Lactobacillus salivarius* est un mutant génétiquement modifié.

37. Utilisation selon une quelconque revendication précédente dans laquelle le *Lactobacillus salivarius* est une variante de *Lactobacillus salivarius* présente dans la nature.

38. Utilisation selon une quelconque revendication précédente dans laquelle le *Lactobacillus salivarius* est sous la forme de cellules viables.

39. Utilisation selon une quelconque revendication précédente dans laquelle le *Lactobacillus salivarius* est sous la forme de cellules non viables.
